# EUROPÄISCHE PATENTSCHRIFT

(11) **EP 1 068 345 B1**
(45) Veröffentlichungstag und Bekanntmachung des Hinweises auf die Patenterteilung: **08.10.2003**
(21) Anmeldenummer: 99923385.1
(22) Anmeldetag: 18.03.1999
(51) Int. Cl.: C12P 19/30, C07H 19/10, C07H 19/20

(54) **NUCLEOTIDAKTIVIERTE DI- UND OLIGOSACCHARIDE SOWIE VERFAHREN ZU DEREN HERSTELLUNG**
NUCLEOTIDE ACTIVATED DISACCHARIDES AND OLIGOSACCHARIDES AND A METHOD FOR THE PRODUCTION THEREOF
DISACCHARIDES ET OLIGOSACCHARIDES A ACTIVATION NUCLEOTIDIQUE ET LEUR PROCEDE DE PREPARATION

(30) Priorität: 20.03.1998 DE 19812156
(43) Veröffentlichungstag der Anmeldung: 17.01.2001
(73) Patentinhaber: Forschungszentrum Jülich GmbH, 52425 Jülich (DE)
(72) Erfinder: ZERVOSEN, Astrid, B-4840 Welkenraedt (BE); ELLING, Lothar, D-52066 Aachen (DE); NIEDER, Veronika, D-52428 Jülich (DE); KAMERLING, Johannis, P., NL-3461 JE Linschoten (NL); VLIEGENTHART, Johannes, F., G., NL-3981 GX Bunnik (NL); GUTIERREZ GALLEGO, Ricardo, NL-3513 CN Utrecht (NL)
(86) Internationale Anmeldenummer: DE9900849
(87) Internationale Veröffentlichungsnummer: WO99047694

(56) Entgegenhaltungen:
- WO-A-94/29477
- HARTMANN, EVAMARIE; KOENIG, HELMUT: "Nucleotide-activated oligosaccharides are intermediates of the cell wall polysaccharide of Methanosarcina barkeri" BIOL. CHEM. HOPPE-SEYLER, Bd. 372, Nr. 11, 1991, Seiten 971-4, XP002112516
- DENAMUR, ROBERT; GAYE, PIERRE: "Isolation and identification of four UDP trisaccharides in the ewe mammary gland and colostrum" EUR. J. BIOCHEM., Bd. 19, Nr. 1, 1971, Seiten 23-35, XP002112517
- HASHIMOTO, HIRONOBU; ENDO, TSUYOSHI; KAJIHARA, YASUHIRO: "Synthesis of the First Tricomponent Bisubstrate Analog That Exhibits Potent Inhibition against GlcNAc:.beta.-1,4-Galactosyltransferase" J. ORG. CHEM., Bd. 62, Nr. 7, 1997, Seiten 1914-5, XP002112518
- SAUER, F. D.; BLACKWELL, B. A.; KRAMER, J. K. G.; MARSDEN, B. J.: "Isolation and characterization of the carbohydrate moiety bound to N-7-mercaptoheptanoyl-O-threonine phosphate" FEMS MICROBIOL. REV. , Bd. 87, 1990, Seiten 333-8, XP002112519
- HEEMSKERK, JOHAN W. M.; STORZ, THOMAS; SCHMIDT, RICHARD R.; HEINZ, ERNST: "Biosynthesis of digalactosyldiacylglycerol in plastids from 16:3 and 18:3 plants" PLANT PHYSIOL. , Bd. 93, Nr. 4, 1990, Seiten 1286-94, XP002112520

## Beschreibung

Die Erfindung betrifft Nucleotidactivierte Di- und Oligosaccharide sowie ein Verfahren zu deren Herstellung.

Glycanstrukturen auf Glycokonjugaten, wie z.B. Glycoproteine und Glycolipide, haben vielfältige biologische Funktionen in der intra- und intermolekularen Kommunikation, die in Übersichtsartikeln von Drickamer 1994 Molecular Glycobilogy Oxford University Press, Oxford; 53-87 ; Feizi 1991, Nature 314, 84-86; Hakomori 1989, Adv. Cancer Res. 52, 257-331; Hart 1992, Curr.Opin.Cell biol. 4, 1017-1023; Lasky 1995, Ann. Rev. Biochem. 64, 113-139. Lis und Sharon 1993, Eur.J. Biochem. 218, 1-27; Rademacher et al. 1988, Ann. Rev. Biochem. 57, 785-838; Varki 1993, Glycobiol. 3, 97-130; Varki 1994, Proc. Natl. Acad. Sci USA 91, 7390.7397 zusammengefaßt worden sind. Die bekanntesten Beispiele sind die Blutgruppenantigene AB0(H), die Lewis-Antigene (Le^{a,b,x,y}) die u.a. in sialylierter Form an der Zell-Zell-Adhäsion als Ligand von E-, P- und L-Selectin beteiligt sind sowie das Galα(1-3)Gal-Epitop, das eine enorme immunologische Barriere bei der Xenotransplantation von tierischen Organen in den Menschen darstellt.

Die Biosynthese der Glycane von Glycoproteinen und Glycolipiden in Eukaryonten (z.B. Säugetiere) ist in einer Reihe von Übersichtsartikeln von Abeijon und Hirschberg 1992, TIBS 17, 31-36; Brockhausen 1995, New. Comp. Biochem Vol 29a, Elsevier Science, Amsterdam, 201-260, Hakomori 1986, Spektrum der Wissenschaften 90-100; Lehle and Tanner 1995, Glycoproteins, New Comp. Biochem Vol 29a Elsevier Science Amsterdam, 201-260; Verbert 1995 Glycoprotiens, New Comp. Biochem Vol 29a, Elsevier Science, Amsterdam, 145-152 ausführlich dokumentiert. In der N-Glycan-Biosynthese der eukayontischer Glycoproteine wird zunächst ein Dolicholdiphosphatoligosaccharid durch sequentiellen Transfer von Monosacchariden aus Nucleotid- oder Dolicholphosphat aktivierten Zukkern aufgebaut, das dann "en bloc" auf ein Protein übertragen wird. O-Glycane der Glycoproteine bzw. der Glycane der Glycosphingolipide werden durch sequentiellen Transfer von Monosacchariden direkt auf die Protein- bzw. Lipidstruktur aufgebaut. Als Substrate dienen hier nucleotidaktivierte Monosaccharide. An der Biosynthese der N- und O-Glycane sind Leioir-Glycosyltransferasen und im Fall der N-Glycane auch Glycosidasen beteiligt. Der Leloir-Biosyntheseweg zum Aufbau von glycosylierten Verbindungen ist in Syntheseweg 1 dargestellt.

Die Veröffentlichung im J. Org. Chem. 1997, 62, 1914-1915 offenbart einen nucleophilen Glycosyl-Acceptor (GlcNAcβ-Ome) für die β-1,4-Galactosyltransferase welcher diese Glycosyltransferase inhibiert.

In der Veröffentlichung Eur. J. Biochem. 19 (1971), 23 offenbart, daß Nucleotid aktivierte Di- und Oligosaccharide Substrate für β-Galactosidase sein können.

Die Veröffentlichung Biol. Chem. Hoppe-Seyler, 372, 971 (1991) postuliert einen Biosyntheseweg für nucleotidaktivierte Di- und Oligosaccharide in Methanosarcina barkeri

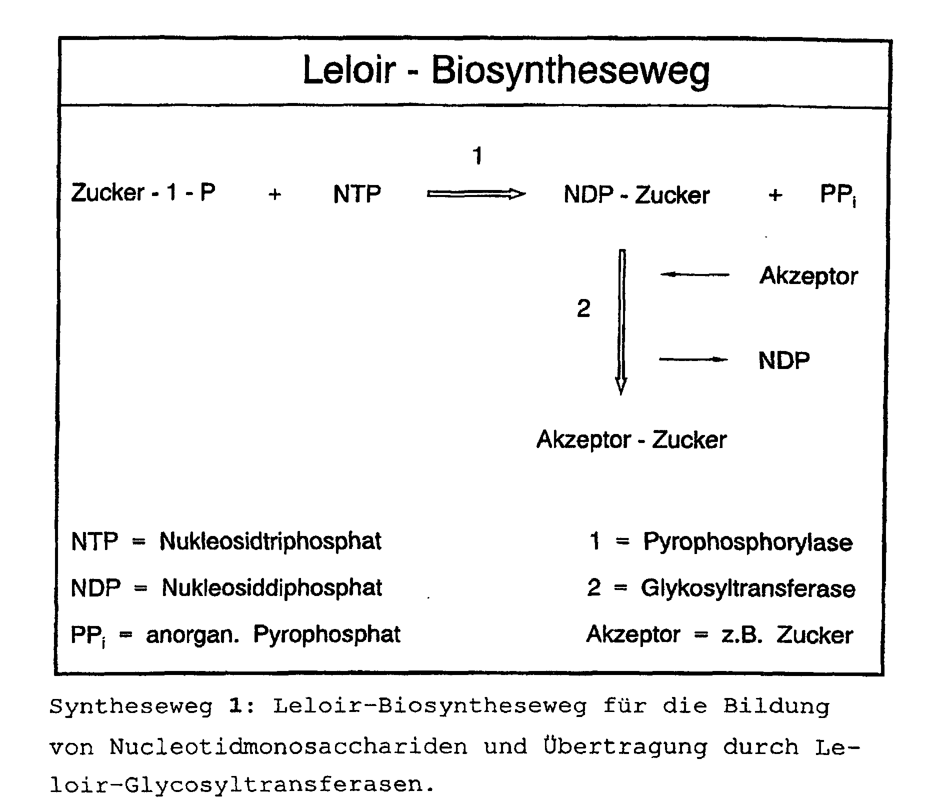

Gemäß Reaktionsschritt 1 katalysieren Pyrophosphorylasen die Synthese von nucleotidaktivierten Monosacchariden, indem sie einen Nucleotidylrest von einem Nucleosidtriphosphat auf einen am anomeren C-Atom phosphorylierten Zucker unter Freisetzung von Pyrophosphat übertragen. Die entstehenden Nucleotidmonosaccharide dienen im Reaktionsschritt 2 als Donorsubstrate von Leloir-Glycosyltransferasen, die den Zucker auf ein Akzeptorsubstrat unter Freisetzung von Nucleosiddiphosphat übertragen. Akzeptorsubstrate können Aglycone sein oder im Fall der Glycanbiosynthese Mono- oder Oligosaccharide.

In Arbeiten von Hindsgaul et al. 1995,J.Carbohydr. Chem. 14 453-464 sowie Srivastava et al. 1992, J. Biol. Chem. 267, 22356-22361 sind bereits modifizierte Trisaccharide chemisch hergestellt und als Donorsubstrate für die α1-3/4Fucosyltransferase aus Humanmilch eingesetzt worden. Dies wurde auch von Crawley und Palcic 1996 in Modern methods in carbohydrate synthesis (Frontiers in natural product research ser. Vol.1, Harwood Academ. Publichers, Amsterdam 492-517 berichtet. Es konnte dabei gezeigt werden, daß der Transfer von größeren Resten, wie z.B. Biotin oder die Trisaccharide der Blutgruppen A und B, auf LacNAc von einer GDPaktivierten und C6-modifizierten Fucose möglich ist. In diesen chemisch hergestellten Fucosederivaten sind die größeren Reste über einen längeren Spacer an die Fucose gebunden.

Ein Problem bei der Entwicklung präparativer Synthesen von Oligosacchariden mit Leloir-Glycosyltransferasen ist die Verfügbarkeit dieser schwer isolierbaren Enzyme. Inzwischen wurden, wie von Elling 1997 in Advances in Biochem. Engineering/Biotechnology 58, 89-144 veröffentlicht wurde, bereits zahlreiche Transferasen kloniert und exprimiert, aber bis heute sind nur wenige Enzyme in ausreichenden Mengen verfügbar. Auch sind 1 heute wichtige Glycosyltransferasen wie z.B. die Mucin β1,3-Galactosyltransferase, die den Transfer von Gal aus UDP-Gal auf α-D-GalNAc-OR katalysiert, nicht zugänglich. Aus diesem Grund wurde das Disaccharid Gal(β1-3)GalNAc bisher entweder in einer aufwendigen Vielstufensynthese oder durch enzymatische Transglycosylierung mit β-Galactosidasen aus Rinderhoden hergestellt, wie von Kren und Thiem 1995 in Angew. Chem. 107, 979-981, sowie Hedbys et al 1989 in Carbohydr. Res. 186, 217-223 gezeigt wurde.
Glycosidasen kataylsieren *in vivo* die Hydrolyse einer glycosidischen Bindung. Man unterscheidet zwischen den Endoglycosidasen, die glycosidische Bindungen in Oligound Polysacchariden hydrolysieren, und den Exoglycosidasen, die Monosaccharide vom nichtreduzierenden Ende einer Oligo- oder Polysaccharidkette abspalten. Glycosidasen können auch zur Synthese von Glycosiden genutzt werden. Dabei wird der Glycosylrest eines Donors (Glycon) in einer thermodynamisch (reverse Hydrolyse) oder einer kinetisch (Transglycosylierung) geführten Reaktion auf die Hydroxylgruppe eines Akzeptors (R-OH, R=Aglycon) stereoselektiv übertragen (Formelbild 1). Glycosidasen arbeiten nicht absolut regioselektiv, so daß Regioisomere als Produkte entstehen. Im weiteren Verlauf der Reaktion kann das entstandene Produkt noch weiter glykosyliert werden. Im allgemeinen werden, wie von Wong and Whitesides 1994 in Enzymes in synthetic Organic Chjemistry, Elsevier Science, Oxfrd, 252-298 berichtet wurde, Ausbeuten von 10% - 40% in von Glycosidasen katalysierten Reaktionen erreicht.

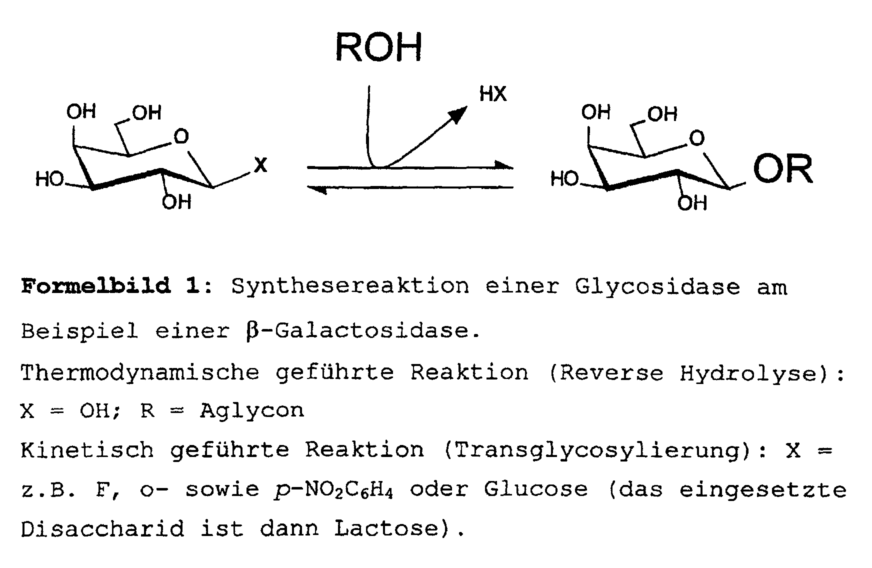

Glycosidasen zeichnen sich durch eine hohe Spezifität im Hinblick auf das Glycon aus. Hingegen ermöglicht ihre geringe Aglyconspezifität die Synthese einer Vielzahl von glycosidisch verknüpften Verbindungen, die in der Veröffentlichung von Nilsson 1996 Modern Methods in Carbohydrate Synthesis - (Frontiers in Natural Product Research Series; Vol. 1), Harwood Academic Pubishers, Amsterdam, 518-547 gezeigt werden. Die Vielzahl der Synthesen mit verschiedenen Exoglycosidasen sind in einer Reihe von Übersichtsartikeln zusammengefaßt worden (Fernandez-Mayorales 1997 Topics in Corrent Chemistry 186, 2-20, Nilsson 1988 Trends in Biotechnol. 6, 256-264, Nilsson 1996 Modern Methods in Carbohydrate Synthesis (Frontiers in natural Product Research Ser. Vol.1, Harwood Academic Publishers, Amterdam, 518-547); Toone et al. 1989, Tetradedron 45, 5365-5422, Wong und Whitesides 1994, Enzymes in Synthetic Organ. Chem., Elsevier Science, Oxford 252-297; Wong et al. 1995 Angew. Chem. 107, 569-593,).
Ein wichtiger Aspekt für die enzymatische Synthese von nucleotidaktivierten Di- und Oligosacchariden ist die Optimierung der Syntheseausbeute, da Nucleotidzucker sehr teure Substrate sind. Es ist daher für die Wirtschaftlichkeit der Synthesen von entscheidender Bedeutung, daß diese Substrate möglichst vollständig umgesetzt oder verbleibendes Substrat zurückgewonnen wird. Im Hinblick auf die Synthesen mit Glycosidasen wurden in der Literatur bereits zahlreiche Synthesemethoden beschrieben, die eine Ausbeutesteigerung zur Folge haben.
So zeigten Vetere und Paoletti 1996 in Biochem. Biophys. Res, Commun. 219, 6-13, daß bei einer Synthese von Galb(1,4)GlcNAc (LacNAc) die Ausbeute von 23 % auf 42 % und die Regioselektivität von 90 % auf 98 % durch Absenkung der Temperatur von 30°C auf 15°C gesteigert werden kann. Bei einer Synthese von Gal(β1-6)GlcNAc unter Katalyse der β-Galactosidase aus *E*. *coli* wurde von Hänsler und Jakubke (1996 Amino Acids 11, 379-395) durch Einfrieren des Reaktionsgemischs bei -5°C eine Ausbeutesteigerung von 35 % (25°C) auf 53 % erzielt.

Es ist die Aufgabe der Erfindung ein neues Verfahren zur Herstellung von Nucleotidaktivierten Di- und Oligosacchariden zu schaffen, welches bessere Ausbeuten an Produkt liefert sowie neue Glycanstrukturen zur Verfügung zu stellen.

Die Aufgabe wird erfindungsgemäß dadurch gelöst, daß ein Nucleotidactiviertes Saccharid mit n Saccharid-Einheiten, wobei n = 2 bis Oligo ist durch eine enzymatische Reaktion von einem Zucker mit einer Abgangsgruppe am anomeren Kohlenstoffatom als Donor mit einem Nucleotidsaccharid mit n-1 Saccharid-Einheiten als Akzeptor umgesetzt wird. Die Umsetzung kann dabei mit einer α- oder β- Glycosidase erfolgen. Vorzugsweise ist die Glycosidase eukaryontischen oder prokaryontischen Ursprungs und vorzugsweise rekombinant.
In einer spezielleren bevorzugten Ausführungsform der Erfindung wird eine β-Galactosidase aus Bacillus circulans oder aus Rinderhoden eingesetzt.
Unter einem Oligosaccharid im Sinne der Erfindung ist ein Saccharid mit n Einheiten zu verstehen, wobei n mindestens 2, aber auch 3,4,5,6,7,8,9,10 ist und nach oberen Werten eine Anzahl von beipsielsweise 20 umfaßt. Jedoch soll nicht zwingend eine Begrenzung auf 20 bestehen, vielmehr soll eine frei wählbare Anzahl von Saccharideinheiten umfaßt werden, die der Fachmann noch nicht unter dem Begriff Polysaccharid versteht. Jedenfalls soll die Anzahl n nicht auf den für die Bezeichnung oligo typischen Bereich von n = 3 oder 10 Einheiten eingeschränkt sein.

Erfindungsgemäß können alle Pyrimidin- und Purinnucleotid-Saccharide mit n-1 Saccharid-Einheiten als Akzetoren eingesetzt werden. Dazu zählen Nucleotidsaccharide vom UDP-, ADP-, CDP-, DTDP-, GDP-,CMP- und IDP-Typ sowie deren Derivate, wie beispielsweise Azido-Derivate. Weitere erfindungsgemäß einsetzbare Nucleotidsaccharide sind Derivate des N-acetylglucosamins bzw. des Glucosamins, der Galactose und deren Derivate sowie des N-acetyl-Galactosamins und dessen Derivate.
Als Donor können die am anomeren Kohlenstoffatom substituierten Derivate aller Zucker eingesetzt werden. Der Substituent am anomeren Kohlenstoff ist dabei eine Abgangsgruppe. Als Abgangsgruppe können beispielhaft Halogenid, insbesondere F⁻, aber auch Cl⁻, Br⁻ und J⁻, Arylalkohole, Substituierte Arylalkohole, Alkenylalkohole und die natürlichen Di- und Oligosaccharidsubstrate der jeweils eingesetzten Glycosidasen genannt werden.

Erfindungsgemäß konnte gezeigt werden, daß Nucleotidmono- Di- und Oligosaccharide als Akzeptorsubstrat für Glycosidasen fungieren können. Bei der Reaktion kann ein Produktgemisch von Nucleotissacchariden aus n und n+1 Saccharid-Einheiten gebildet werden.
Mit dem erfindungsgemäßen Verfahren können Nucleotidaktivierte Di- und Oligosaccharide dargestellt werden, welche pro Molekül verschiedene Zuckereinheiten aufweisen. Für die verschiedenen Reaktionsschritte müssen dann unterschiedliche Glycosidasen eingesetzt werden.

Im folgenden soll die Erfindung beispielhaft erläutert werden:
Syntheseweg 2 zeigt, daß beispielsweise mit der β-Galactosidase (EC 3.2.1.23) aus *Bacillus circulans* Gal(β1-4)GlcNAc(α1-UDP und Gal(β1-4)Glc(α1-UDP synthetisiert werden können (Beispiel 1-4). Mit der β-Galactosidase aus Rinderhoden kann Gal(β1-3)GalNAc(α1-UDP und und Regioisomere synthestisiert werden Beispiel 5) (Syntheseweg 3)

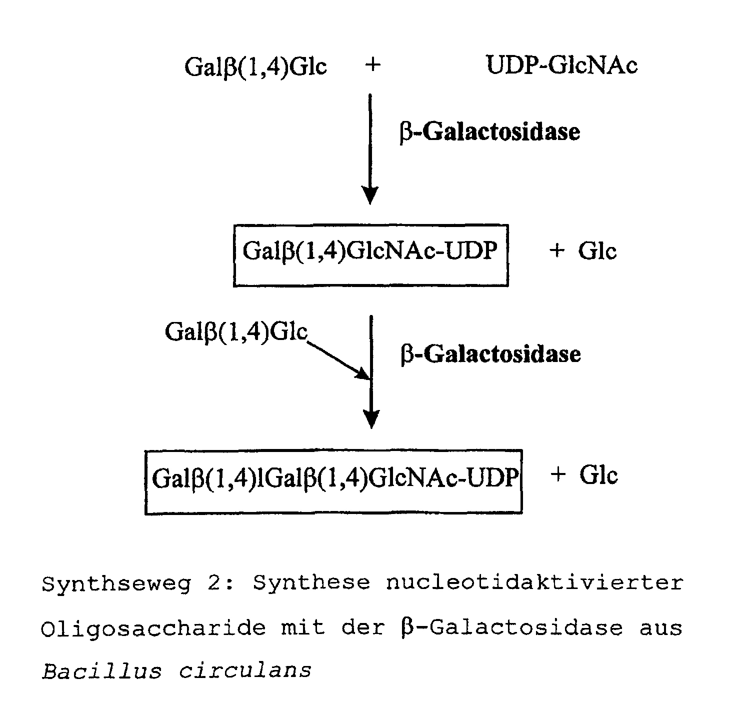

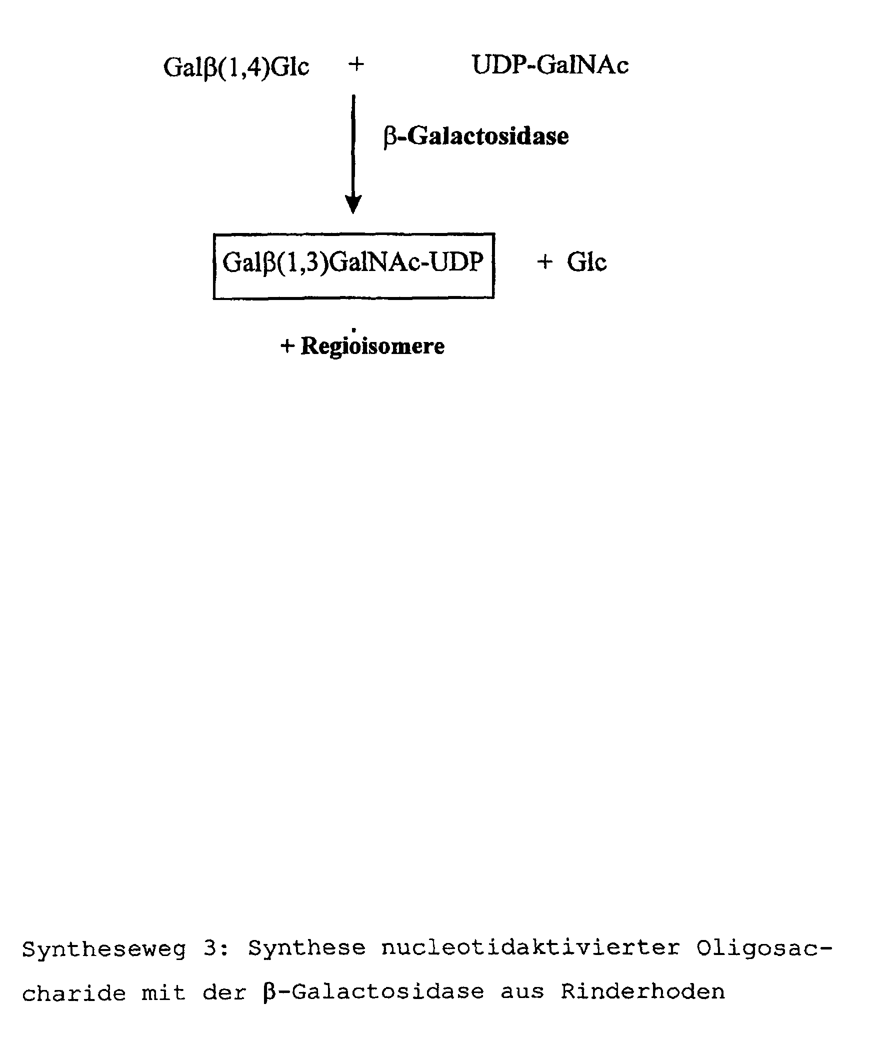

Die im Beispiel 1 beschriebenen Tieftemperatursynthesen zeigen, daß β-Galactosidase aus *Bacillus circulans* bei -5°C aktiv ist und daß die Ausbeuten der Synthese von Gal(βl-4)GlcNAc(α1-UDP von 24% (30°C) auf 34% (-5°C) und von Gal(β1-4)Gal(βl-4)GlcNAc(al-UDP von 6.5% (30°C) auf 10% (-5°C) gesteigert wurden. Im Anschluß an die Synthese kann der nicht umgesetzte Nucleotidzucker mit der beschriebenen Isolierungsmethode zurückgewonnen werden, so daß durch die Kombination der Tieftemperatursynthese mit der entwickelten Produktisolierung eine wirtschaftliche Synthesemethode entwickelt wurde.

Das Nutzungspotential von nucleotidaktivierten Di- und Oligosacchariden als neuartige Verbindungen liegen in folgenden Bereichen:
1) Sie können als Standards in HPLC-Analysen von Zellextrakten dienen, um die Biosynthese nucleotidaktivierter Disaccharide (z.B. UDP-LacNAc) nachzuweisen.
2) Nucleotidaktivierte Di- und Oligosaccharide können als Akzeptorsubstrate für weitere Leloir-Glycosyltransferasen oder Glycosidasen beim Aufbau von Neoglycokonjugaten dienen. Das am reduzierende Ende befindliche Nucleotid kann dabei als UV-Marker in der HPLC-Analytik oder zur Anbindung an einen Ionenaustauscher genutzt werden.
3) Ein sehr attraktiver Aspekt ist, nucleotidaktivierte Di- und Oligosaccharide als Donorsubstrate für Leloir-Glycosyltransferasen zu nutzen. Damit könnte ein enzymatischer 'en bloc' Transfer von Di- und Oligosacchariden auf einen Akzeptor *in vitro* etabliert werden.
4) Nucleotidaktivierte Di- und Oligosaccharide könnten auch potentielle Inhibitoren von Leioir-Glycosyltransferasen und Nucleotidzuckertransportern darstellen.

### Anwendungsbeispiele:

Die im folgenden beschriebenen Methoden wurden für alle Beispiele angewendet:

### Material und Methoden für die MS und NMR Analysen

### Massenspektrometrie

Die Molmassen der Reaktionsprodukte wurden mittels Fast Atom Bombardment Mass Spectrometry (FAB MS) im Negativ Ionen Modus an einem JEOL JMS-SX/SX 102A Viersektoreninstrument bei einer Beschleunigungsspannung von 6 keV durchgeführt. Das Gerät war mit einer JEOL MS-FAB 10D FAB gun ausgerüstet, die mit einem 10 mA Emissionstrom arbeitet und einen Strahl von 4 keV Xenon Atome erzeugt. 1.0 µl Probe (10 µg in 0,1 ml 5% Essigsäure) wurde gelöst, in 1,0 µl Glycerin oder Thioglycerin Matrix dispergiert und lineare Massenabtastungen über 1000 Dalton aufgezeichnet. Die aufgezeichneten Daten wurden mit der JEOL Komplement Software ausgewertet (Bijvoet Center, Department of Mass Spectrometry).

### NMR Spektroskopie

Die Reaktionsprodukte wurden vor der Analyse wiederholt in D₂O (99,9 Atom % D; Isotec) aufgenommen und gefriergetrocknet und schließlich in 450 µl D₂O (99,96 Atom %D, Isotec) gelöst. Proton-entkoppelte 75.469-MHz ¹³C NMR Spektren wurden an einem Bruker AC-300 Spektrometer bei Probentemperaturen von 300 K aufgezeichnet. Chemische Verschiebungen (δ) beziehen sich auf externes Aceton (δ 31,08). Auflösungsverstärkte ¹H 1D and 2D NMR Spektren wurden an Bruker AMX-500 oder Bruker AMX-600 Instrumenten (Department of NMR spectroscopy, Utrecht University) bei Probentemperaturen von 300 K aufgenommen. Chemische Verschiebungen (δ) wurden in ppm bezogen auf internes Acetat (δ 1,908) ausgedrückt. HOD Signalunterdrückung wurde durch Anwendung einer WEFT Pulssequenz [K. Hard, G. van Zadelhoff, P. Moonen, J.P. Kamerling, and J.F.G. Vliegenthart, Eur. J. Biochem., 209 (1992) 895-915.] in 1D ¹H Experimenten und durch Vorsättigung für 1 s in 2D Experimenten erreicht.
2D TOCSY Spektren wurden mit Hilfe von MLEV-17 Mischungssequenzen mit effektiven Spin-Lock Zeiten zwischen 20 und 100 ms aufgezeichnet. 2D ROESY Spektren wurden mit einer Mischungszeit von 250 ms aufgezeichnet. Die Spin-Lock Feldstärke entsprach einem 90° Puls von ca. 115 µs. Proton ermittelnde ¹³C-¹H 2D HMBC Experimente wurden bei einer ¹H Frequenz von 500,139 und 600,140 MHz (125,769 und 150,916 MHz für ¹³C) mit einer von Summers et al. [M.F.Summers, L:G: Marzilli, und C. Whifield, J. Biol. Chem., 272 (1997) 4121-4128] beschriebenen Pulssequenz durchgeführt. Die Verzögerungszeit für die Ermittlung der long-range ¹³C-¹H Kopplungen wurde auf 60 ms festgesetzt. ¹H 1D und 2D Spektren wurden an Silicon Graphics IRIS Workstationen (Indigo 2 and 02)mit Bruker UXNMR Software (Bijvoet Center, Department of NMR spectroscopy) ausgewertet. ¹³C 1D Spektren wurden an Silicon Graphics IRIS Workstationen (Indigo 2 and 02) mit TRITON Software (Bijvoet Center, Department of NMR spectroscopy) bearbeitet.

Den Anwendungsbeispielen sind folgende Figuren zugehörig:
Fig.1: HPLC-Chromatogramm einer Synthese von Gal(β1-4)GlcNAc(α1-UDP (UDP-LacNAc) und Gal(β1-4)Gal(β1-4)GlcNAc(α1-UDP mit der β-Galactosidase aus *Bacillus circulans*.
Fig.2: Ausbeute-Zeit-Kurven einer Synthese von Gal(β1-4)GlcNAc(α1-UDP und Gal(β1-4)Gal(β1-4)GlcNAc(α1-UDP mit der β-Galactosidase aus *Bacillus circulans*
Fig.3 a und b: DC-Chromatogramme der Synthesen mit verschiedener aktivierter Nucleotidzucker mit β-Galactosidase aus *Bacillus circulans*
Fig. 3c-f: HPLC-Chromatogramme der Reaktionsprodukte aus einer Reaktion mit β-Galactosidase aus *Bacillus circulans*.
Fig.4: Ausbeute-Zeit-Kurven der Synthesen verschiedener aktivierter Nucleotidzucker mit der β-Galactosidase aus *Bacillus circulans*.
Fig.5: FAB MS (Negativ Ionen Modus, Glycerin) des UDP-LacNAc's, das mit β-Galactosidase aus *Bacillus circulans* synthetisiert wurde.
Fig.6: 1D ¹H 500-MHz NMR Spektrum des UDP-LacNAc's, das mit β-Galactosidase aus *Bacillus circulans* synthetisiert wurde, bei 300K.
Fig.7: 1D ¹³C 75-MHz NMR Spektrum des UDP-LacNAc's, das mit β-Galactosidase aus *Bacillus circulans* synthetisiert wurde, bei 300K.
Fig.8: FAB MS (Negativ Ionen Modus, Glycerin) der UDP-Lactose, die mit β-Glactosidase aus *Bacillus circulans* synthetisiert wurde.
Fig.9: 1D ¹H 500-MHz NMR Spektrum der UDP-Lactose, das mit β-Galactosidase aus *Bacillus circulans* synthetisiert wurde, bei 300K.
Fig.10: 1D ¹³C 75-MHz NMR Spektrum der UDP-Lactose, die mit β-Galactosidase aus *Bacillus circulans* synthetisiert wurde, bei 300K.
Fig.11 a und b: Synthese von Gal(β1-3)GalNAc(α1-UDP mit der β-Galactosidase aus Rinderhoden (HPLC-Chromatogramm)
Fig.12: Struktur von Gal(β1-4)Glc[NAc](α1-UDP

### Beispiel 1:

### Synthese von Gal(β1-4)GlcNAc(α1-UDP (UDP-LacNAc) und Gal(β1-4)Gal(β1-4)GlcNAc(α1-UDP mit β-Galactosidase (EC 3.2.1.23) aus Bacillus circulans bei 30°C und -5°C

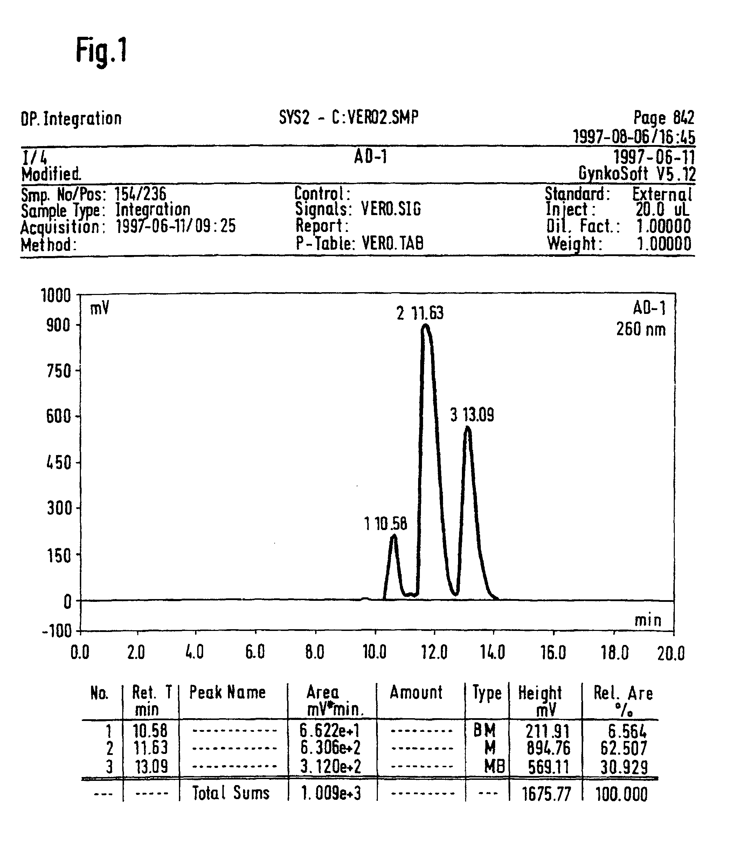

500 mM Lactose und 100 mM UDP-GlcNAc wurden mit 10 U/ml β-Galactosidase (Handelsname: Biolacta N5) in 20 mM KH₂PO₄-Puffer pH 4.5 gelöst und nach Schockgefrieren in flüssigem Stickstoff bei -5°C inkubiert. Bei einer Temperatur von 30°C wurde der Ansatz in 20 mM Natriumacetatpuffer pH 7.0 inkubiert. Die pH-Werte wurden jeweils bei 25°C eingestellt. Die Reaktion wurde durch ein kurzzeitiges Erhitzen (5 min.) auf 95°C abgestoppt und mittels Ionenpaar-Reverse-Phase-HPLC (Elling, L. and Kula, M.-R. (1993) J. Biotechnology **29**, 277-286). analysiert. Bei der Reaktion wurde sowohl ein nucleotidaktiviertes Disaccharid Gal(β1-4)GlcNAc(1α-UDP (Gal₁(1-4)GlcNAc-UDP) als auch ein aktiviertes Trisaccharid Gal(β1-4)Gal(β1-4)GlcNAc(α1-UDP (Gal₂(1-4)GlcNAc-UDP) gebildet (Fig.1)
Fig. 1 zeigt ein HPLC-Chromatogramm einer Synthese von Gal(β1-4)GlcNAc(α1-UDP (UDP-LacNAc) und Gal(β1-4)Gal(β1-4)GlcNAc(α1-UDP mit der β-Galactosidase aus *Bacillus circulans.*
10.58 min. Gal(β1-4)Gal(β1-4)GlcNAc(α1-UDP, 11.63 min. UDP-GlcNAc, 13.09 min. Gal(β1-4)GlcNAc (α1-UDP

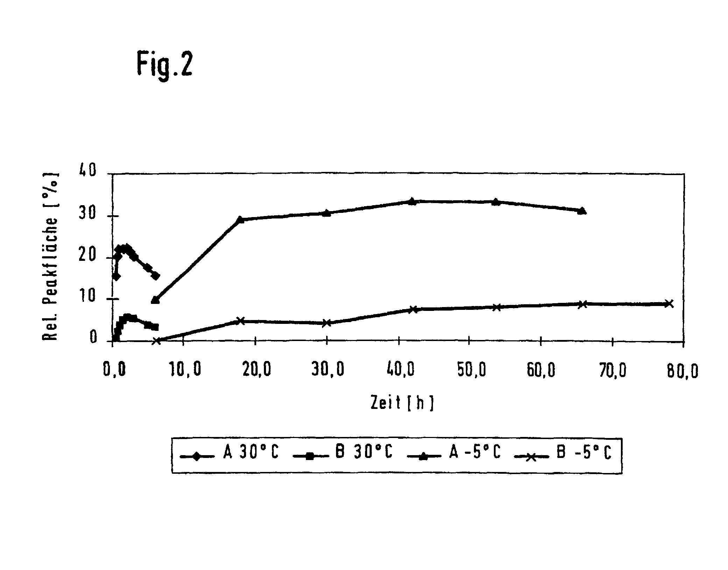

Fig. 2 zeigt Ausbeute-Zeit-Kurven einer Synthese von Gal(β1-4)GlcNAc(α1-UDP und Gal(β1-4)Gal(β1-4)GlcNAc(α1-UDP mit der β-Galactosidase aus *Bacillus circulans* Reaktionsbedingungen: 500 mM Lactose, 100 mM UDP-GlcNAc, 10 U/ml β-Galactosidase, Puffer: 20 mM KH₂PO₄ pH 4.5 bei -5°C und 20 mM Natriumacetatpuffer pH 7.0 bei 30°C
A: Gal(β1-4)GlcNAc(α1-UDP; B: Gal(β1-4)Gal(β(1-4)GlcNAc(α1-UDP
Die Ausbeute-Zeit-Kurven in Fig.2 zeigen, daß die Ausbeuten bei -5°C deutlich erhöht sind. Hier wurde eine maximale Ausbeute von 34% Gal₁(1-4)GlcNAc-UDP nach 1.75 Tagen (24% bei 30 °C nach 90 Minuten) und von 10% Gal₂(1-4)GlcNAc-UDP nach 3.75 Tagen (6.5% bei 30°C nach 120 Minuten) erreicht. Bei -5°C konnte im Gegensatz zur Synthese bei 30°C keine Hydrolyse im Synthesezeitraum von 3.5 Tagen beobachtet werden.

### Beispiel 2:

### Synthesen aktivierter Oligosaccharide mit der β-Galactosidase (EC 3.2.1.23) aus Bacillus circulans

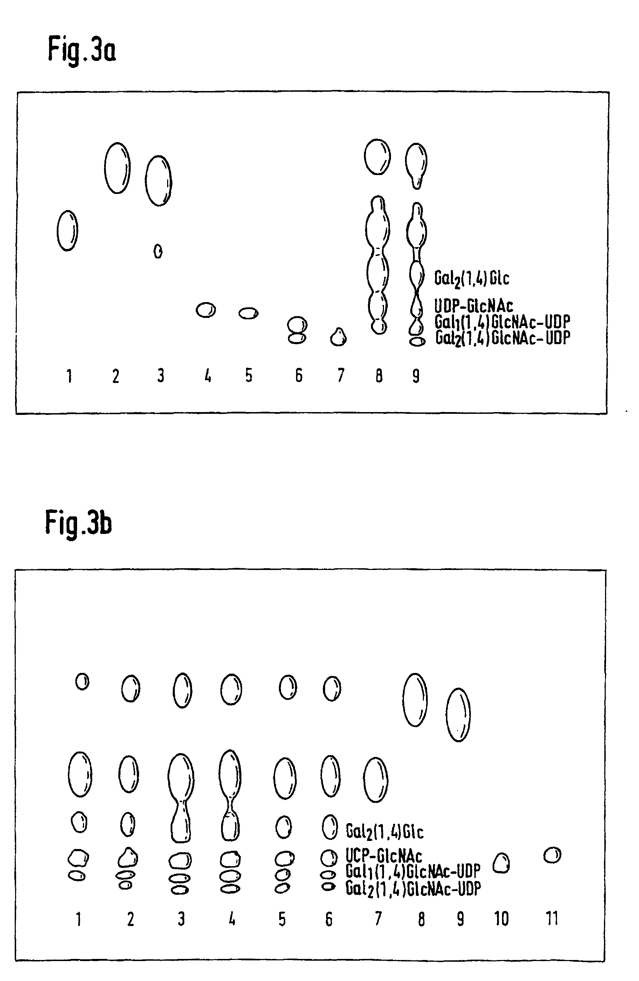

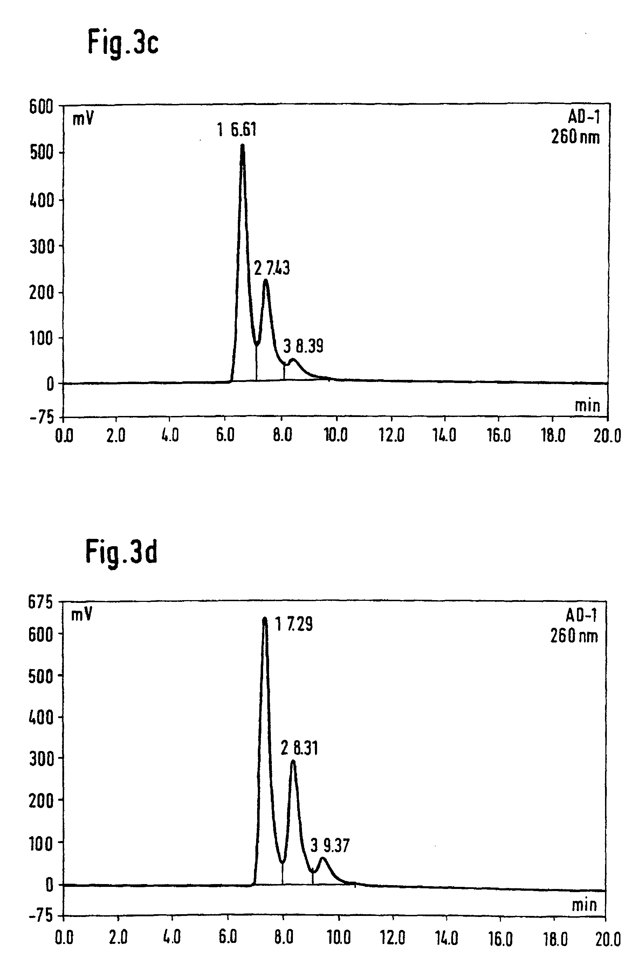

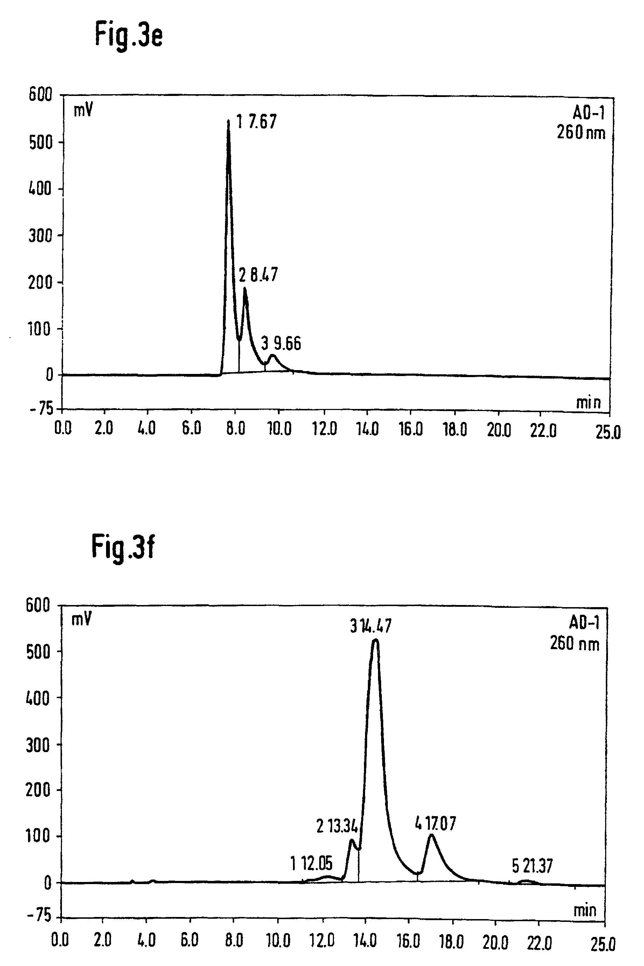

500 mM Lactose wurden mit 100 mM UDP-Glc, 100 mM UDP-Gal bzw. 100 mM UDP-GalNAc mit 10 U/ml β-Galactosidase in 20 mM KH₂PO₄-Puffer pH 4.5 nach Schockgefrieren in flüssigem Stickstoff bei -5°C inkubiert. Der pH-Wert wurde bei 25°C eingestellt. Die Reaktion wurde durch ein kurzzeitiges Erhitzen (5 min) auf 95°C abgestoppt und mittels Ionenpaar-Reverse-Phase-HPLC bzw. (Elling, L. and Kula, M.-R. (1993) J. Biotechnology **29,** 277-286.) DC-Analytik (Trägermaterial: Kieselgel60 _{F254} ; Laufmittel: Acetonitril (75 %): 0.1 M NH₄Cl (25 %) (Peters et al. 1993); Anfärbemittel: 20 mg Naphthoresorcinol, 40 mg Diphenylamin, 10 ml Ethanol, 400 µl H₂SO_{4 konz}) analysiert. Bei der Reaktion wurde sowohl aktivierte Disaccharide Gal₁(1-4)X-UDP als auch aktivierte Trisaccharide Gal₂(1-4)X-UDP (X = Glc, Gal, Gal-NAc) gebildet (Fig.3).
Die Figuren 3a und b zeigen DC-Chromatogramme der Synthesen mit verschiedenen aktivierten Nucleotidzucker mit β-Galactosidase aus *Bacillus circulans.*
Reaktionsbedingungen: 500 mM Lactose, 100 mM UDPaktiviertes Monosaccharid, 10 U/ml β-Galactosidase, 20 mM KH₂PO₄ pH 4.5 bei -5°C, Inkubationszeit: 2 Tage Fig 3a: Dünnschichtchromatographische Trennung des Reaktionsansatzes mit UDP-GalNAc als Akzeptorsubstrat:
1. Lactose (Standard)
2. Glucose (Standard)
3. Galactose (Standard)
4. UDP-GlcNAc (Standard)
5. UDP-GalNAc (Standard)
6. Gal₂(1-4)GlcNac-UDP + Gal₁(1-4)GlcNAc-UDP
7. Gal₂(1-4)GlcNAc-UDP
8. Reaktionsansatz UDP-GalNAc
9. Reaktionsansatz UDP-GlcNac (als Vergleich)

Fig. 3b. Dünnschichchromatographische Trennung des Reaktionsansatzes mit UDP-Gal als Akzeptorsubstrat
1. Reaktionsansatz mit UDP-Gal (0,25 Tage)
2. Reaktionsansatz mit UDP-Gal (0,75 Tage)
3. Reaktionsansatz mit UDP-Gal (1,25 Tage)
4. Reaktionsansatz mit UDP-Gal (1,75 Tage)
5. Reaktionsansatz mit UDP-GlcNAc (1,25 Tage) (Vergleich)
6. Reaktionsansatz mit UDP-GlcNAc (1,75 Tage) (Vergleich)
7. Lactose (Standard)
8. Glucose (Standard)
9. Galactose (Standard)
10. UDP-Gal (Standard)
11. UDP-GlcNAc (Standard)

Figuren 3c,d,e und f:
HPLC-Chromatogamme der von der β-Galactosidase aus B. *circulans* katalysierten Reaktion von UDP-GlcNAc, UDP-GalNAc. Die Reaktionen (Ansatzgröße 15 µl) wurden mit 500 mM Lactose, 100 mM des jeweiligen Nucleotidzuckers und 10 U/ml β-Galactosidase in 20 mM NaAc Puffer (pH 4.5) bei -5°C durchgeführt. Die Reaktionsdauer betrug 2 Tage.
HPLC-Säule: Nucleosil 100-5 (Macherey &Nagel) Fluß: 1 ml/min
Laufmittel: Acetonitril: 0,1 M NH₄Cl (70:30) für UDP-GlcNAc und UDP-Gal
Acetonitril: 0,1 M NH₄Cl (75:25) für UDP-GalNac

Fig. 3c: UDP-Glc als Akzeptor:
UDP-Glc (6,61 min), Gal₁(1-4)Glc-UDP (7,43min.) und Gal₂(1-4)Glc-UDP (8,39 min)

Fig. 3d: UDP-GlcNAc als Akzeptor:
UDP-Glc-NAC (7,29 min), Gal₁(1-4)GlcNAc-UDP ( 8,31 min) und Gal₂GlcNAc-UDP (9,37 min)

Fig.3e: UDP-Gal:
UDP-Gal (7,67 min), Gal₁(1-4)Gal-UDP (8,47 min,) und Gal₂(1-4)Gal-UDP (9,66 min)

Fig.3f: UDP-GalNAc:
UDP-GalNAc (14,47 min), Gal₁(1-4)GalNAc-UDP (17,07 min.)

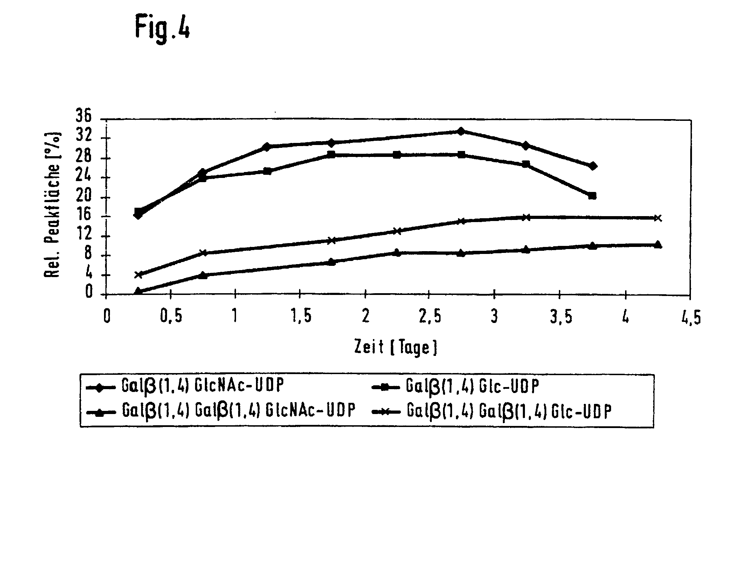

Fig.4 zeigt Ausbeute-Zeit-Kurven der Synthesen verschiedener aktivierter Nucleotidzucker mit der β-Galactosidase aus *Bacillus circulans.*
Reaktionsbedingungen: 500 mM Lactose, 100 mM UDP-GlcNAc bzw. UDP-Glc, 10 U/ml β-Galactosidase, 20 mM KH₂PO₄ pH 4.5 bei -5°C

Die Ausbeute-Zeit-Kurve in Fig. 4 verdeutlicht, daß unter den gewählten Synthesebedingungen auch mit dem Akzeptor UDP-Glc im Vergleich mit UDP-GlcNAc gute Ausbeuten an aktiviertem Di- und Trisaccharid erzielt wurden.

### Beispiel 3:

### Präparative Synthese von Gal(β1-4)GlcNAc(α1-UDP mit β-Galactosidase (EC 3.2.1.23) aus Bacillus circulans

500 mM Lactose wurden mit 100 mM UDP-GlcNAc (Na₂-Salz) und 10 U/ml β-Galactosidase in 20 mM Puffer (Natriumacetat/Essigsäure, pH 4.5) zwei Tage bei -5°C inkubiert. Das Volumen des Ansatzes war 2 ml. Das Abstoppen der Reaktion erfolgte durch Hitzedenaturierung des Enzyms 5 min. lang bei 95°C. Die Syntheseausbeute betrug 22.8% (bezogen auf den Akzeptor) an Gal(β1-4)GlcNAc(α1-UDP. Die Analytik erfolgte mittels Ionenpaar-Reverse-Phase-HPLC (Elling und Kula 1993 J. Biotechnol. 29, 277-286).

Die Produktisolierung erfolgte in 4 Schritten:
1) Abtrennung von Protein über eine Ultrafiltrationsmembran Microcon 10 von Amicon (Mitten) in der Tischzentrifuge Biofuge A von Heraeus Christ (Osterode) bei 12000 rpm.
2) Die ungeladenen Zucker und der Puffer wurden durch Anionenaustauschchromatographie abgetrennt. Die Chromatographieanlage bestand aus einer Säule (Innendurchmesser: 2.6 cm; Betthöhe: 16.5 cm) mit 88 ml Sepharose Q FF, einer Pumpe P-1, einem UV-Detektor (UV-1) mit einer S-2 UV-Durchflußzelle (254 nm), einer Leitfähigkeitsmeßzelle und einem Schreiber. Alle Materialien waren von Pharmacia (Freiburg). Die Säule wurde durchgehend mit einer Flußrate von 4 ml/min betrieben. Die Säule wurde mit dem dreifachen Säulenvolumen entionisiertem Wasser equilibriert. Der Syntheseansatz wurde auf die Säule geladen, die anschließend mit entionisiertem Wasser (eingestellt auf pH 2, dreifaches Säulenvolumen) gespült wurde. Die Elution der Nucleotidzucker erfolgte mit einer 0.4 M NaCl-Lösung (pH 2.0). Die Fraktionen, die Nucleotidzukker enthielten (HPLC-Analytik, Elling und Kula 1993 J. Biotechnol. 29, 277-286) wurden vereinigt und der pH-Wert wurde mit NaOH auf pH 7.0 eingestellt. Die Säule konnte dann mit folgendem Spülprogramm vollständig regeneriert werden: dreifaches Säulenvolumen 1 M NaCl, dreifaches Säulenvolumen entionisiertes Wasser. Das Säulenmaterial wurde in 20%iger Ethanollösung gelagert.
3) Der Pool aus der Sepharose Q Trennung wurde im Wasserstrahlvakuum bei 35°C auf maximal 4 ml eingeengt. Die hohe NaCl-Konzentration in der Lösung, die durch die Elution von der Sepharose Q FF-Säule resultiert, wurde durch Fällung mit Ethanol (50 %) bei Raumtemperatur weitgehend minimiert. Festes NaCl wurde abzentrifugiert und das Ethanol wurde im Wasserstrahlvakuum bei 35°C entfernt. Die Ethanolfällung wurde zweimal durchgeführt.
4) Eine präparative Gelfiltration mit Biogel-P2 extra fine Material von Biorad München wurde bei Raumtemperatur durchgeführt. Es wurden 1 ml Fraktionen gesammelt. Die Auftrennung des Reaktionsgemisches wurde mittels Ionenpaar-Reverse-Phase-HPLC bzw. ( Elling, L. and Kula, M.-R. (1993) J. Biotechnology **29**, 277-286.) DC-Analytik (Trägermaterial: Kieselgel60 _{F254} ; Laufmittel: Acetonitril (75 %) : 0.1M NH₄Cl (25 %) (Peters et al. 1993 Tetrahedr. Asym. 4, 1173-1182); Anfärbemittel: 20 mg Naphthoresorcinol, 40 mg Diphenylamin, 10 ml Ethanol, 400 µl H₂SO_{4 konz}) verfolgt. Eine vollständige Isolierung des Produktes war nur nach mehrfacher Gelfiltration möglich. Dazu wurde der Pool jeweils auf 1.5 ml eingeengt und nach einem Zusatz von NaCl (Salzkonzentration in der Probe: 0.1 mmol), erneut auf der P2-Säule gereinigt. Abschließend wurde die Lösung lyophilisiert.

Insgesamt wurden 6.8 mg Gal(β1-4)GlcNAc(α1-UDP (Na₂-Salz) mit einer Gesamtausbeute von 4.2% (100% Reinheit laut HPLC-Analyse) isoliert. Das Produkt wurde durch NMR-Spektroskopie und Massenspektrometrie charakterisiert.

Charakterisierung des Produktes Gal(β1-4)GlcNAc(α1-UDP synthetisiert mit β-Galactosidase aus *Bacillus circulans*

### Massenspektrometrie

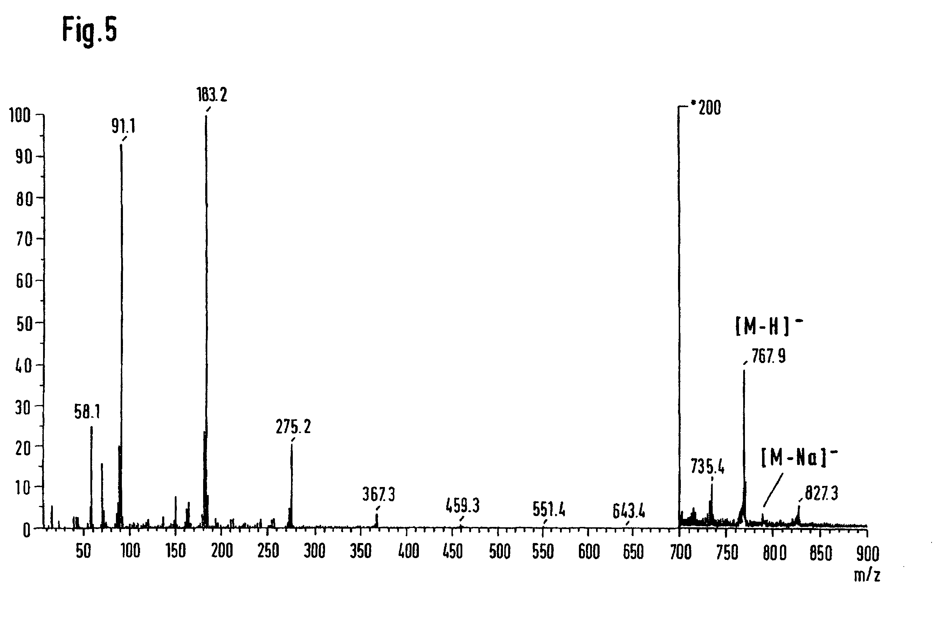

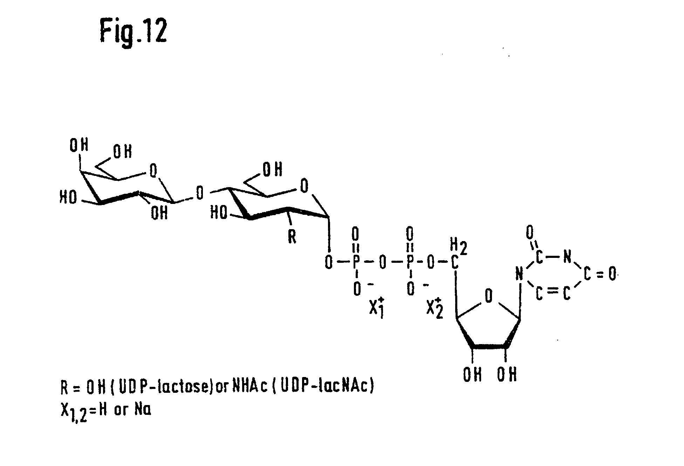

Das FAB MS (Negativ Ionen Modus, Glycerin) Spektrum (Figur 5: FAB MS ( negativer Ionenmodus, Glycerol) von UDP-LacNAc, hergestellt mit β-Galactosidase aus *Bacillus circulans)* des Reaktionsproduktes zeigte ein typisches Glycerin Spektrum (*m*/*z* [91 + n.92] mit n = 0,1,2...9) mit einem sehr starken Peak in der Hochmassenregion bei *m*/*z* 767,9, der dem [M-H]⁻ pseudomolekularen Ion der Struktur (X_{1,2} = H) in Figur 12 entspricht.

### NMR Spektroskopie

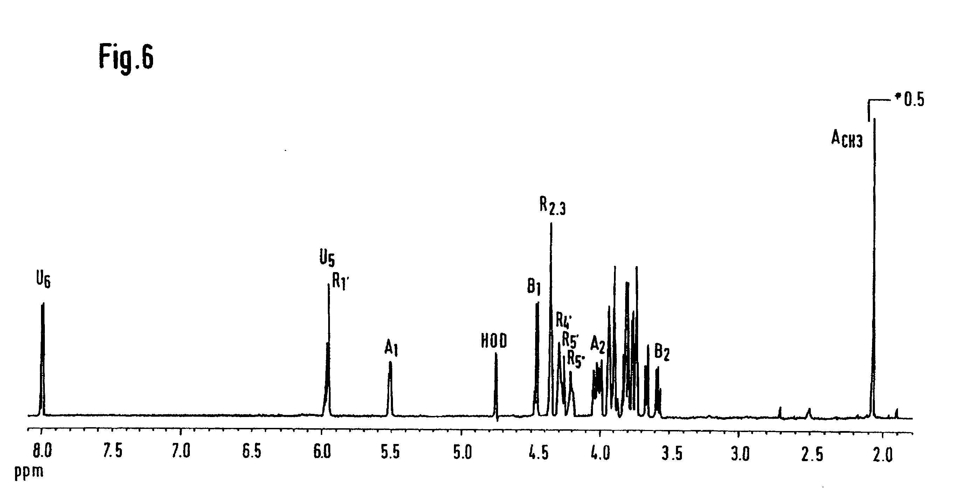

Das 1D ¹H NMR Spektrum (Figur 6: 1D ¹H 500-MHz NMR-Spektrum von UDP-LacNAc mit β-Galactosidase aus *Bacillus circulans*, bei 300 K) zeigte vier Signale im Tieffeld vom HOD Signal (δ 4,751). Zwei dieser Signale, bei δ 7,998 (³*J*_{6,5} 8,4 Hz, U₆) and δ 5,962 (³*J*_{5,6} 8,4 Hz, U₅) wurden den Protonen des Uracilrings an Hand von Literaturdaten zugeordnet [ R. Koplin, J.-R. Brisson, and C. Whitfield, J. Biol. Chem., 272 (1997) 4121-4128. J.-H. Yoon and K. Ajisaka, Carbohydr. Res., 292 (1996) 153-163.
Das anomere Signal bei δ 5,952 (³*J*_{1,2} 3,6 Hz) wurde dem Riboserest R (β Konfiguration, Furanoseringform) zugeordnet, wohingegen das verbleibende anomere Signal bei δ 5,510 (³*J*_{1,2} 3,0 Hz, ³*J*_{1,P} 7,2 Hz) dem GlcNAc-Rest A (α Konfiguration, Pyranoseringform), der mit der Phosphatgruppe verbunden ist, zugeordnet wurde. Zwei chemische Verschiebungen im Hochfeld von der HOD Resonanz konnten auch zugeordnet werden; das Singlet bei δ 2,071, das von den N-acetyl Protonen des GlcNAc's A stammt sowie das anomere Doublet bei δ 4,458 (³*J*_{1,2} 7,8 Hz), das den Gal-Rest B reflektiert (β Konfiguration, Pyranoseringform).

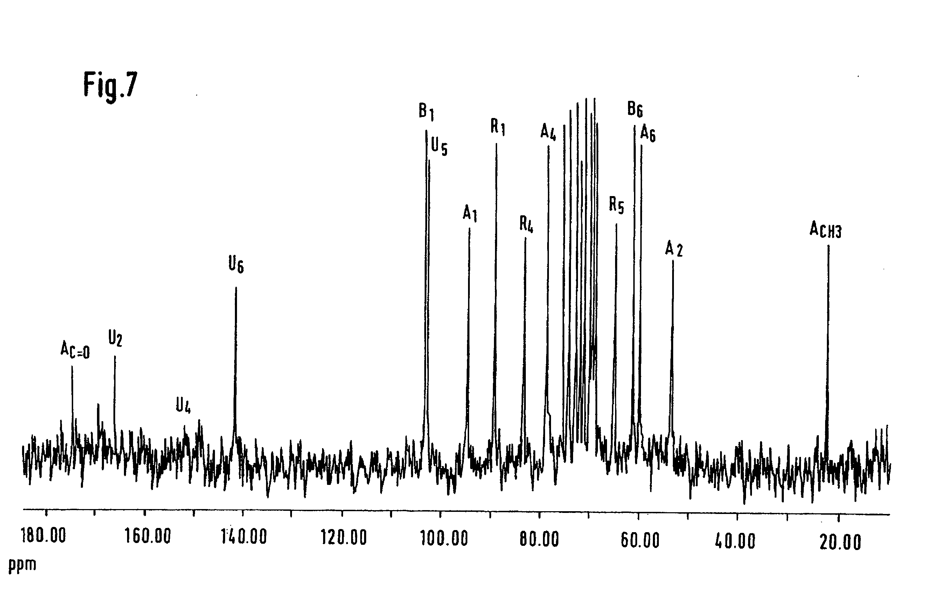

Das ¹³C NMR Spektrum (Figur 7 1D ¹³C 75-MHz NMR-Spekrum von UDP-LacNAc, hergestellt mit β-Glactosidase aus *Bacillus circulans*, bei 300 K ) zeigte vier Signale in der anomeren Region bei δ 103,80 (B₁), 103,21 (U₅), 95,04 (A₁) und 89,58 (R₁). Weiterhin wurden vier Signale in der Tieffeldregion bei δ 175,41 (A_{C=O}), 166,92 (U₂), 152,10 (U₄) und 142,02 (U₆) beobachtet. Typische GlcNAc Resonanzen wurden bei δ 22,77 (NAc-CH₃) und δ 53,81 (A₂) gefunden. Die Signale bei δ 60,29, 61,68 und 65,39 spiegeln die Anwesenheit von drei Hydroxymethylgruppen wider, von denen die letztere substituiert ist.

Durch 2D TOCSY, ROESY und HMBC Experimente konnten alle ¹³C und ¹H Resonanzen in den 1D Spektren zugeordnet werden (Tabelle 1).

**Tabelle 1:**

| ¹H and ¹³C NMR chemische Verschiebungen des UDP-LacNAc's, das mit β-Galactosidase aus *Bacillus circulans* synthetisiert wurde (aufgenommen bei 300 K). Die Kopplungskonstanten (Hz) sind in Klammern angegeben. | | |
|---|---|---|
| Rest | UDP-LacNAc ¹H^{a} | ¹³C^{b} |
| A-1 | 5.510 (3.0,7.2) | 95.04 |
| A-2 | 4.039 | 53.81 |
| A-3 | 3.813 | 70.41 |
| A-4 | 3.786 | 78.98 |
| A-5 | 4.013 | 72.41 |
| A-6a | 3.779^{c} | 60.29 |
| A-6b | 3.906^{c} | - |
| A CH₃ | 2.071 | 22.77 |
| A C=O | - | 175.4 1 |
| | | |
| B-1 | 4.458 (7.8) | 103.8 0 |
| B-2 | 3.566 | 71.57 |
| B-3 | 3.648 | 73.25 |
| B-4 | 3.925 | 69.34 |
| B-5 | 3.722 | 76.04 |
| B-6a | 3.850^{c} | 61.68 |
| B-6b | 3.791^{c} | - |
| | | |
| R-1 | 5.952 | 89.58 |
| | (3.6) | |
| R-2 | 4.358 | 74.75 |
| R-3 | 4.358 | 69.82 |
| R-4 | 4.296 | 83.65 |
| R-5a | 4.261 | 65.39 |
| R-5b | 4.210 | - |
| | | |
| U-2 | - | 166.9 2 |
| | | |
| U-4 | - | 152.1 0 |
| | | |
| U-5 | 5.962 (8.4) | 103.2 1 |
| | | |
| U-6 | 7.998 (8.4) | 142.0 2 |

| | | |
|---|---|---|
| ^{a} In ppm relativ zum Signal von internem Acetat. | | |
| ^{b} In ppm relativ zum Signal von externem Aceton. | | |
| ^{c} Die Zuordnung von H-6a und H-6b könnte innerhalb eines Restes untereinander ausgetauscht werden. | | |

### Beispiel 4:

### Präparative Synthese von Gal(β1-4)Glc(α1-UDP (UDP-Lactose) mit β-Galactosidase aus Bacillus circulans

500 mM Lactose wurden mit 100 mM UDP-Glc (Na₂-Salz) und 10 U/ml β-Galactosidase in 20 mM Puffer (Natriumacetat/Essigsäure, pH 4.5) zwei Tage bei -5°C inkubiert. Das Volumen des Ansatzes war 2 ml. Das Abstoppen der Reaktion erfolgte durch Hitzedenaturierung des Enzyms 5 min. lang bei 95°C. Die Syntheseausbeute betrug 24.5% (bezogen auf den Akzeptor) an Gal(β1-4)Glc(1α-UDP. Die Analytik erfolgte mittels Ionenpaar-Reverse-Phase-HPLC (Elling, L. and Kula, M.-R. (1993) J. Biotechnology 29, 277-286.).
Die Produktisolierung erfolgte in 4 Schritten analog zur Isolierung von Gal(β1-4)GlcNAc(α1-UDP (Beispiel 3). Insgesamt wurden 10.2 mg Gal(β1-4)Glc(α1-UDP mit einer Gesamtausbeute von 6.1% (92.6% Reinheit laut HPLC-Analyse) isoliert. Das Produkt wurde durch NMR-Spektroskopie und Massenspektrometrie charakterisiert.

Charakterisierung des Produktes Gal(β1-4)Glc(α1-UDP synthetisiert mit β-Galactosidase aus *Bacillus circulans*

### Massenspektrometrie

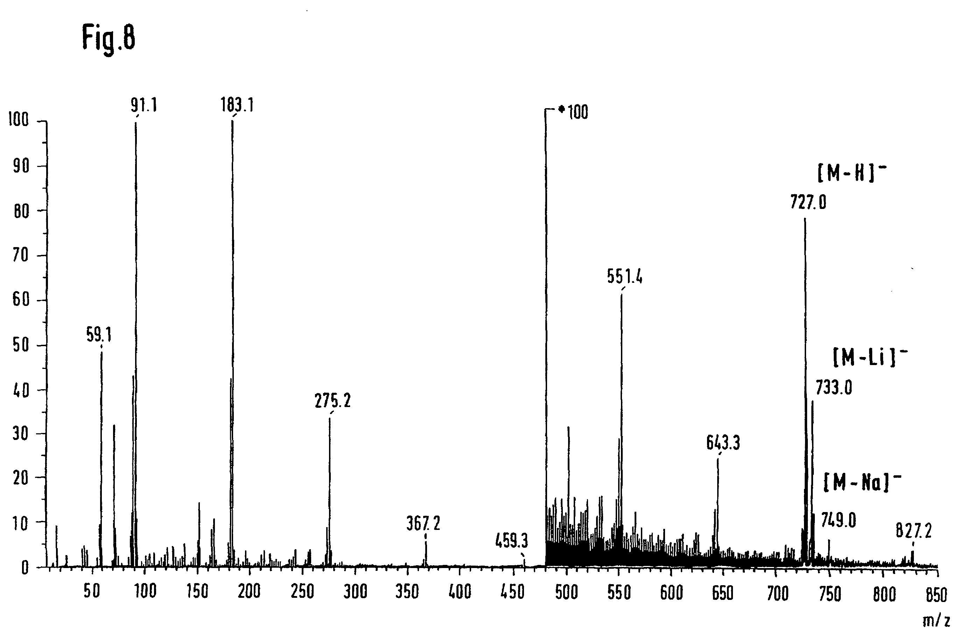

Der stärkste Peak in der Hochmassenregion des FAB MS (Negativ Ionen Modus, Glycerin) Spektrums (Figur 8 FAB MS (negativer Ionen-Modus, Glycerol) von UDP-Lactose, hergestellt mit Galactosidase) des Reaktionsproduktes bei *m*/*z* 727,0 entspricht dem [M-H]⁻ pseudomolekularen Ion der Struktur (X_{1,2} = H) in Figur 12. Das Natrium Analoge (X_{1,2} = Na) wurde bei *m*/*z* 749,0 ([M-Na]⁻) beobachtet. Ein zusätzliches Signal bei *m*/*z* 733,0 wurde der Lithium-Form ([M-Li]⁻, X_{1,2} = Li) zugeordnet. Die verbleibenden beobachteten Signale stammen von Matrixclusterionen. Es wurde keine signifikante Fragmentierung beobachtet.

### NMR Spektroskopie

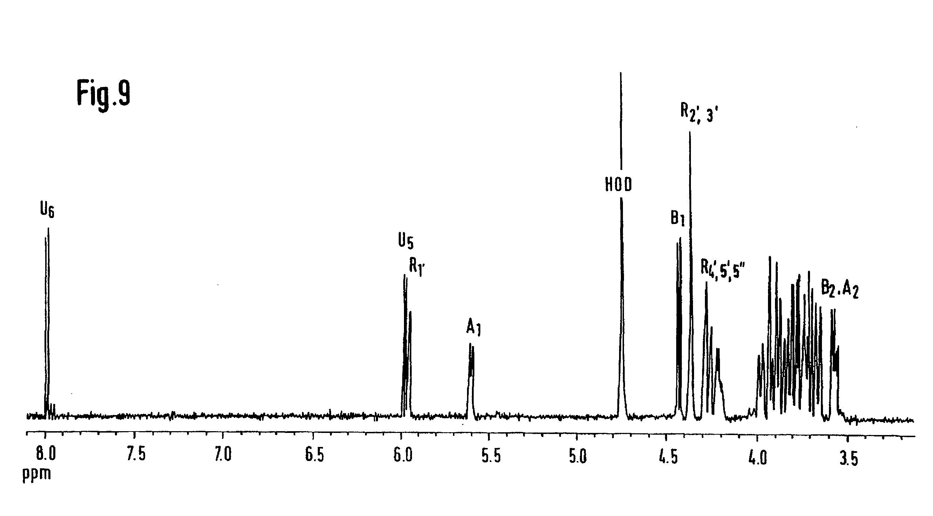

Das 1D ¹H NMR Spektrum (Figur 9 1D ¹H 5 MHz NMR-Spektrum von UDP-Lactose, hergestellt mit Galactosidase, bei 300K) zeigte vier Signale im Tieffeld vom HOD Signal (δ 4,751). Zwei dieser Signale, bei δ 7,998 (³*J*_{6,5} 8,4 Hz, U₆) and δ 5,977 (³*J*_{5,6} 8,4 Hz, U₅) wurden den Protonen des Uracilrings an Hand von Literaturdaten zugeordnet [R. Koplin, J.-R. Brisson C. Whitfield, J. Biol. Chem. 272,(1997) 4121-2128; J.-H. Yoon, K. Ajisaka, Carbohydr. Res., 292 (1996) 153-163]. Das anomere Signal bei δ 5,958 (³*J*_{1,2} 3,5 Hz) wurde dem Riboserest R (β Konfiguration, Furanoseringform) zugeordnet, wohingegen das verbleibende anomere Signal bei δ 5,596 (³*J*_{1,2} 3,5 Hz, ³*J*_{1,P} 7,1 Hz) dem Glc-Rest A (α Konfiguration, Pyranoseringform), der mit der Phosphatgruppe verbunden ist, zugeordnet wurde. Das anomere Doublet, im Hochfeld der HOD Resonanz, bei δ 4,455 (³*J*_{1,2} 7,5 Hz) reflektiert den Gal-Rest B (β Konfiguration, Pyranoseringform).

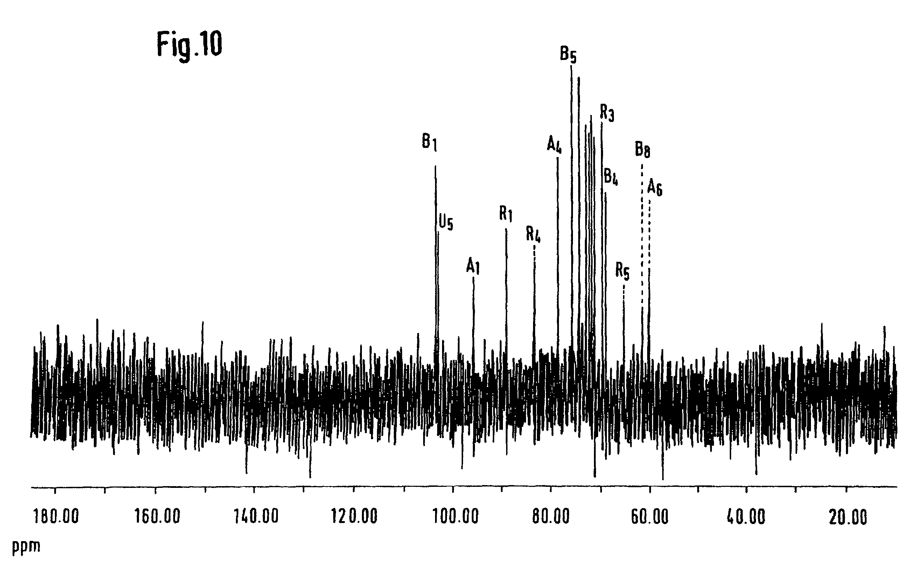

Das ¹³C NMR Spektrum (Figur 10 1D ¹³C 75- MHz NMR-Spektrum von UDP-Lactose, hergestellt mit Galactosidase, bei 300K) zeigte vier Signale in der anomeren Region bei δ 104,12 (B₁), 103,31 (U₅), 96,53 (A₁) und 89,67 (R₁). Weiterhin wurden drei Signale in der Tieffeldregion bei δ 166,40 (U₂), 152,75 (U₄) und 141,93 (U₆) beobachtet. Die Signale bei δ 60,26, 61,68 und 65,38 spiegeln die Anwesenheit von drei Hydroxymethylgruppen wider, von denen die letztere substituiert ist.

Durch 2D TOCSY, ROESY und HMBC Experimente konnten alle ¹³C und ¹H Resonanzen in den 1D Spektren zugeordnet werden (Tabelle 2).

**Tabelle 2:**

| ¹H and ¹³C NMR chemische Verschiebungen der UDP-Lactose, die mit β-Galactosidase aus *Bacillus circulans* synthetisiert wurde (aufgenommen bei 300 K). Die Kopplungskonstanten (Hz) sind in Klammern angegeben. | | |
|---|---|---|
| Rest | UDP-Lac ¹H^{a} | ¹³C^{b} |
| A-1 | 5.596 (3.5, 7.1) | 96.53 |
| A-2 | 3.565 | 72.45 |
| A-3 | 3.795 | 72.56 |
| A-4 | 3.687 | 78.92 |
| A-5 | 3.980 | 72.14 |
| A-6a | 3.885^{c} | 60.26 |
| A-6b | 3.906^{c} | - |
| | | |
| B-1 | 4.455 (7.5) | 104.1 2 |
| B-2 | 3.576 | 71.88 |
| B-3 | 3.665 | 73.31 |
| B-4 | 3.933 | 69.17 |
| B-5 | 3.722 | 76.33 |
| B-6a | 3.845^{c} | 61.68 |
| B-6b | 3.799^{c} | - |
| | | |
| R-1 | 5.958 (3.5) | 89.67 |
| R-2 | 4.358 | 74.85 |
| R-3 | 4.370 | 69.92 |
| R-4 | 4.299 | 84.13 |
| R-5a | 4.281 | 65.38 |
| R-5b | 4.266 | - |
| | | |
| U-2 | - | 166.4 0 |
| U-4 | - | 152.7 5 |
| U-5 | 5.977 (8.4) | 103.3 1 |
| U-6 | 7.988 (8.4) | 141.9 3 |

| | | |
|---|---|---|
| ^{a} In ppm relativ zum Signal von internem Acetat. | | |
| ^{b} In ppm relativ zum Signal von externem Aceton. | | |
| ^{c} Die Zuordnung von H-6a und H-6b könnte innerhalb eines Restes untereinander ausgetauscht werden. | | |

### Beispiel 5:

### Synthese von Gal(β1-3)GalNAc(α1-UDP mit β-Galactosidase aus Rinderhoden

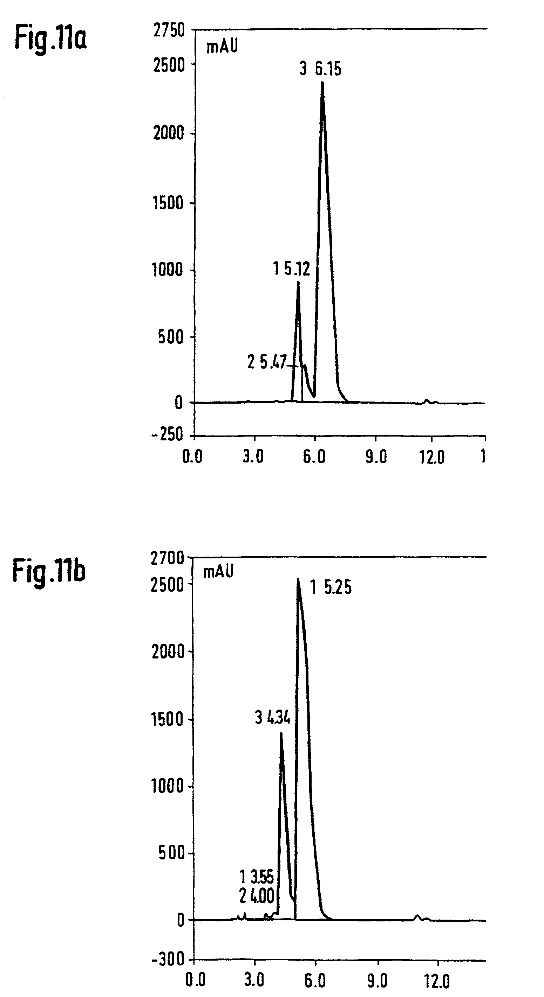

500 mM Lactose wurden mit 100 mM UDP-GalNAc und β-Galactosidase aus Rinderhoden (1.8 U/g Lactose) in 50 mM Phosphat-Citrat-Puffer pH 4.3 mit 0.02 % Natriumazid bei 37°C 24 Stunden inkubiert. Nach 4 und 7 h Inkubationszeit wurden zwei Produktpeaks mit der HPLC-Analytik detektiert. Nach 24 h konnte nur noch ein Produktpeak mit einer relativen Fläche von 20% beobachtet werden (Fig.11).
Fig.11 a: Synthese von Gal(β1-3)GalNAc-UDP mit der β-Galactosidase aus Rinderhoden
7 h: 5.12 min. Gal (β1-3)GalNAc(α1-UDP, 5.47 min. Regioisomer, 6.15 min.UDP-GalNAc
Fig. 11b: 24 h: 4.34 min. Gal(β1-3)GalNAc(α1-UDP + Regioisomer, 5.25 min. UDP-GalNAc
Die hier eingesetzte HPLC-Analytik ist von Ryll,T. (1995) Dissertation TH Braunschweig. Srivastava, G.; Kanwal, J. K.; Hindsgaul, O. and Palcic, M.M. (1992) J. Biol. Chem. **267**; 22356-22361. beschrieben worden.

## Patentansprüche

1. Verfahren zur Herstellung von nucleotidaktivierten Sacchariden mit n Saccharid-Einheiten, wobei n = 2 bis oligo ist, bei dem als Donor ein Zucker mit einer Abgangsgruppe am anomeren Kohlenstoffatom mit einem Nucleotidsaccharid mit n-1 Saccharid-Einheiten als Akzeptor enzymatisch umgesetzt wird.

2. Verfahren nach Anspruch 1,
**dadurch gekennzeichnet,**
**daß** die Umsetzung mittels einer Glycosidase erfolgt.

3. Verfahren nach Anspruch 2,
**dadurch gekennzeichnet,**
**daß** eine β-Galactosidase aus Bacillus circulans oder aus Rinderhoden eingesetzt wird.

4. Verfahren nach einem der Ansprüche 2 oder 3,
**dadurch gekennzeichnet,**
**daß** die Glycosidase eukaryontischen oder prokaryontischen Ursprungs ist.

5. Verfahren nach einem der Ansprüche 2 bis 4,
**dadurch gekennzeichnet,**
**daß** die Glycosidase rekombinant ist.

6. Verfahren nach einem der Ansprüche 1 bis 5,
**dadurch gekennzeichnet,**
**daß** die Umsetzung bei einer Temperatur von -5°C erfolgt.

7. Verfahren nach einem der Ansprüche 1 bis 6,
**dadurch gekennzeichnet,**
**daß** das Nucleotidsaccharid mit n-1 Saccharid-Einheiten eine Komponente aus der Gruppe der Nucleotid-Saccharide, deren Nucleotide vom UDP-, ADP-, CDP-, DTDP-, GDP-, CMP- und IDP-Typ sind, ist.

8. Verfahren nach einem der Ansprüche 1 bis 7,
**dadurch gekennzeichnet,**
**daß** die anomere Abgangsgruppe des als Donor eingesetzten Zuckers eine Komponente aus der Gruppe von Halogen, Aryl, Arylalkohol, Alkenylalkohol, Alkenyl und Glycosyl ist.

9. Verfahren nach einem der Ansprüche 1 bis 8,
**dadurch gekennzeichnet,**
**daß** eine Mischung aus Nucleotidsacchariden mit n und n+1 Saccharid-Einheiten gebildet wird.

10. Verwendung von nucleotidaktivierten Di- oder Oligosacchariden, hergestellt nach dem Verfahren gemäß einem der Ansprüche 1 bis 9, als Standard für die biochemische Analytik.

11. Verwendung von nucleotidaktivierten Di- oder Oligosacchariden als Donorsubstrate für Leloir-Glycosyltransferasen.

## Claims

1. Method for manufacturing nucleotide-activated saccharides with n saccharide units, n being 2 to oligo, wherein a sugar with an initial group on the anomeric carbon atom, as donor, is converted enzymatically with a nucleotide saccharide with n-1 saccharide units as acceptor.

2. Method according to Claim 1, **characterised in that** the conversion is carried out by means of a glycosidase.

3. Method according to Claim 2, **characterised in that** a *β*-galactosidase from bacillus circulans or from bovine testicles is used.

4. Method according to one of Claims 2 or 3, **characterised in that** the glycosidase is of eukaryotic or prokaryotic origin.

5. Method according to one of Claims 2 to 4, **characterised in that** the glycosidase is recombinant.

6. Method according to one of Claims 1 to 5, **characterised in that** the conversion takes place at a temperature of -5°C.

7. Method according to one of Claims 1 to 6, **characterised in that** the nucleotide saccharide with n-1 saccharide units is a component of the group of nucleotide saccharides whose nucleotides are of the UDP, ADP, CDP, DTDP, GDP, CMP and IDF type.

8. Method according to one of Claims 1 to 7, **characterised in that** the anomeric initial group of the sugar used as the donor is a component of the group comprising halogen, aryl, aryl alcohol, alkenyl alcohol, alkenyl and glycosyl.

9. Method according to one of Claims 1 to 8, **characterised in that** a mixture of nucleotide saccharides with n and n+1 saccharide units is formed.

10. Use of nucleotide-activated di- or oligosaccharides, manufactured by the method according to one of Claims 1 to 9, as a standard for the biochemical analysis.

11. Use of nucleotide-activated di- or oligosaccharides as donor substrates for Leloir glycosyl transferases.

## Revendications

1. Procédé de fabrication de saccharides activés par des nucléotides, ayant n unités de saccharides, où n est = 2 à oligo, dans lequel, comme donneur, un sucre comportant un groupe de départ sur l'atome anomère de carbone est transformé de manière enzymatique avec un nucléotide-saccharide ayant n-1 unités de saccharide comme accepteur.

2. Procédé selon la revendication 1,
**caractérisé en ce**
**que** la transformation s'effectue au moyen d'une glycosidase.

3. Procédé selon la revendication 2,
**caractérisé en ce qu'**une β-galactosidase provenant de Bacillus circulans ou de testicules de bovidé est utilisée.

4. Procédé selon l'une des revendications 2 ou 3,
**caractérisé en ce que** la glycosidase est d'origine eucaryote ou protocaryote.

5. Procédé selon l'une des revendications 2 à 4,
**caractérisé en ce que** la glycosidase est recombinante.

6. Procédé selon l'une des revendications 1 à 5,
**caractérisé en ce que** la transformation s'effectue à une température de -5°C.

7. Procédé selon l'une des revendications 1 à 6,
**caractérisé en ce que** le nucléotide-saccharide ayant n-1 unités de saccharides est une composante du groupe des nucléotides-saccharides dont les nucléotides sont de type UDP, ADP, CDP, DTDP, GDP, CMP et IDP.

8. Procédé selon l'une des revendications 1 à 7,
**caractérisé en ce que** le groupe de départ anomère du sucre utilisé comme donneur est une composante du groupe comprenant halogène, aryle, arylalcool, alkénylalcool, alkényle et glycosyle.

9. Procédé selon l'une des revendications 1 à 8,
**caractérisé en ce qu'**un mélange de nucléotides-saccharides ayant n et n+1 unités de saccharide est formé.

10. Utilisation de di ou d'oligosaccharides activés par nucléotides, fabriqués par le procédé selon l'une des revendications 1 à 9, comme standard pour l'analyse biochimique.

11. Utilisation de di ou d'oligosaccharides activés par nucléotides comme substrats de donneur pour glycosyl-transférases de Leloir.
